# EUROPÄISCHE PATENTSCHRIFT

(11) **EP 3 280 478 B1**
(45) Veröffentlichungstag und Bekanntmachung des Hinweises auf die Patenterteilung: **27.02.2019**
(21) Anmeldenummer: 15715257.0
(22) Anmeldetag: 09.04.2015
(51) Int. Cl.: A61M 16/04, A61M 16/10

(54) **DECKEL FÜR EIN GEHÄUSE EINES FEUCHTE-WÄRME-TAUSCHERS**
COVER FOR A HOUSING OF A MOISTURE-HEAT EXCHANGER
COUVERCLE POUR UN BOÎTIER DE NEZ ARTIFICIEL

(43) Veröffentlichungstag der Anmeldung: 14.02.2018
(73) Patentinhaber: Andreas Fahl Medizintechnik-Vertrieb GmbH, 51149 Köln (DE)
(72) Erfinder: FAHL, Andreas, 50676 Köln (DE)
(74) Vertreter: Geskes, Christoph
(86) Internationale Anmeldenummer: PCT/EP2015/057718
(87) Internationale Veröffentlichungsnummer: WO 2016/162065

(56) Entgegenhaltungen:
- EP-A1- 2 236 165
- DE-U1-202013 010 194
- US-A- 4 582 058
- US-A- 5 022 394
- US-A1- 2002 156 527
- US-A1- 2009 050 156
- US-A1- 2010 288 284
- US-A1- 2011 220 108

## Beschreibung

Die Erfindung betrifft einen Deckel mit einem Schild zur Befestigung an dem Deckel für ein Gehäuse eines Feuchte-Wärme-Tauschers für Tracheotomierte und Laryngektomierte, sowie einen Bausatz.

Feuchte-Wärme-Tauscher für tracheotomierte Patienten sind aus dem Stand der Technik allgemein bekannt. Diese erwärmen und befeuchten die Atemluft mittels eines meist in einer Kassette oder Gehäuse angeordneten Filters. Der Filter speichert die Wärme und Feuchtigkeit der ausgeatmeten Luft und führt diese beim Einatmen der Atemluft wieder zu.

Nachteilig an den bekannten Feuchte-Wärme-Tauscher-Gehäusen ist, dass die nur umständlich beziehungsweise aufwendig von eventuellen Aufbauten entfernt werden können. So ist beispielsweise von der Firma Atos Medical die PROVOX HME CAP bekannt, die auf einen Feuchte-Wärme-Tauscher-Filter aufsteckbar ist. Diese soll einen Verschluss des Filters beziehungsweise des Tracheostomas mit dem Finger ermöglichen, so dass ein Sprechen über eine Stimmprothese ermöglicht wird.

Die genannte Aufsteckkappe umfasst einen gewölbten Titanring der starr ausgeführt ist und ein Atemloch aufweist. Nachteilig an der bekannten Kombination aus Filter beziehungsweise Filterkassette und Kappe ist, dass direkt unterhalb der Kappe der Feuchte-Wärme-Tauscher-Filter angeordnet ist. Dies ist zum einen optisch von Nachteil als auch beim Verschließen des Atemlochs für die Einleitung des Sprechvorganges kommt der Benutzer mit dem Finger in direkte Berührung mit dem Filter, was zur Verunreinigung führen kann. Ein weiterer Nachteil der bekannten Feuchte-Wärme-Tauscher ist, dass das Gehäuse nur sehr umständlich von den Aufbauten zu entfernen ist und es somit bei älteren Personen zu Problemen bei der Nutzung kommen kann.

Aus dem Stand der Technik sind auch die folgenden Feuchte-Wärme-Tauscher bekannt, US 5,022,394, US 209/050156.

Aufgabe der Erfindung ist es daher, die bekannten Nachteile aus dem Stand der Technik zu überkommen und einen verbesserten Feuchte-Wärme-Tauscher für Tracheotomierte und Laryngektomierte zur Verfügung zu stellen.

Die Aufgabe wird, nach Anspruch 1, gelöst mittels eines Deckels mit einem Schild zur Befestigung an dem Deckel für ein Gehäuse eines Feuchte-Wärme-Tauschers für Laryngektomierte und Tracheotomierte umfassend eine als weitgehend kreisrunde Scheibe ausgebildete Deckelplatte, einen Deckelrand und eine Anzahl von den Deckel durchgreifende Ausnehmungen zumindest ein teilweise kreisrund umlaufendes erstes Rastelement angeordnet ist, wobei der Deckelrand außenseitig zumindest ein teilweise umlaufendes zweites Rastelement umfasst, wobei der Schild eine Atemöffnung aufweist, wobei der Schild um die Atemöffnung eine trichterförmige Ausgestaltung aufweist, um einen Finger auf das Atemloch bei einem gewollten Verschluss desselben zu führen, wobei der Schild eine zumindest teilweise umlaufende innenseitige Rastaufnahme für das zweite Rastelement des Deckels umfasst. Weiterhin wird die Aufgabe erfindungsgemäß gelöst mittels eines Bausatzes umfassend zumindest eine Anzahl von Gehäusen eine Rastaufnahme zur Aufnahme eines ersten Rastelements eines Deckels aufweisen, eine Anzahl von Deckeln und eine Anzahl von Schilden. Weitere vorteilhafte Ausgestaltungen sind der nachfolgenden Beschreibung, den Figuren sowie den Unteransprüchen zu entnehmen. Die einzelnen Merkmale der verschiedenen Ausgestaltungen sind jedoch nicht auf diese beschränkt sondern können untereinander und mit anderen Merkmalen zur weiteren Ausgestaltung verknüpft werden.

Es wird ein Deckel mit einem Schild zur Befestigung an dem Deckel für ein Gehäuse eines Feuchte-Wärme-Tauschers für Tracheotomierte und Laryngektomierte vorgeschlagen, der eine als weitgehend kreisrunde Scheibe ausgebildete Deckelplatte, einen Deckelrand und eine Anzahl von den Deckel durchgreifenden Ausnehmungen umfasst, wobei die Deckelplatte zwei oder mehr Ausnehmungen aufweist, wobei innenseitig am Deckelrand zumindest ein teilweise kreisrund umlaufendes erstes Rastelement angeordnet ist, wobei der Deckelrand außenseitig zumindest ein teilweise umlaufendes zweites Rastelement umfasst, wobei der Schild eine Atemöffnung aufweist, wobei der Schild um die Atemöffnung eine trichterförmige Ausgestaltung aufweist, um einen Finger auf das Atemloch bei einem gewollten Verschluss desselben zu führen, wobei der Schild eine zumindest teilweise umlaufende innenseitige Rastaufnahme für das zweite Rastelement des Deckels umfasst.

Vorteilhafterweise ist in einer Ausgestaltung vorgesehen, dass der Deckel auf einem weiteren Bauteil eines Feucht-Wärme-Tauschers, insbesondere einem Gehäuse, klemmbar befestigbar ist. Weiterhin ist bevorzugt vorgesehen, dass der Deckel aufschraubbar oder aufrastbar ist. In einer vorteilhaften Ausgestaltung ist vorgesehen, dass der Deckel Klemmelemente aufweist. Vorteil an der Ausgestaltung ist, dass ein sicherer Sitz insbesondere auf dem Gehäuse gewährleistet ist und gleichzeitig die Herstellung des Feucht-Wärmetauschers insbesondere in Hinblick auf die Werkzeugkosten vereinfacht wird.

In einer Ausführungsform ist vorgesehen, dass innenseitig am Deckelrand zumindest ein Rastelement angeordnet ist.

Erfindungsgemäß ist vorgesehen, dass innenseitig am Deckelrand zumindest ein teilweise kreisrund umlaufendes erstes Rastelement angeordnet ist. Gemäß einer Ausführungsform ist das Rastelement als Nut ausgestaltet. Vorzugsweise weist das Gehäuse zumindest ein vorzugsweise ebenfalls kreisrund umlaufend ausgestaltetes Rastelement beziehungsweise eine kreisrund ausgestaltete Deckelaufnahme auf, die mit zumindest dem ersten Rastelement des Deckels zusammenwirkt. Dies hat den Vorteil, dass der Deckel drehbar auf dem Gehäuse angeordnet ist. In einer weiteren Ausgestaltung sind das zumindest eine Rastelement des Gehäuses und das zumindest eine Rastelement des Deckels derart ausgestaltet, dass diese drehhemmend miteinander verrastbar sind. Vorzugsweise ist eine Drehhemmung mittels Kraftschluss vorgesehen.

In einer weiteren Ausführungsform ist vorgesehen, dass innenseitig am Deckelrand eine Anzahl von Rastelementen angeordnet sind. Vorzugsweise sind die Rastelemente als Rastnasen oder Rastvorsprünge ausgestaltet. In einer bevorzugten Ausgestaltung ist vorgesehen, dass die Rastelemente sich etwa 0,1 mm bis etwa 0,9 mm, bevorzugt etwa 0,4 mm bis etwa 0,8 mm radial nach innen über den Deckelrand erheben. Diese Ausgestaltung erlaubt eine besonders kompakte Bauweise.

In einer weiteren Ausgestaltung ist vorgesehen, dass die Rastelemente am Innenumfang des Deckelrandes umlaufend verteilt sind. Vorzugsweise sind etwa 3 bis etwa 20 Rastelemente, weiter bevorzugt etwa 6 bis etwa 15 Rastelemente, weiter bevorzugt 6 oder 8 oder 9 Rastelemente vorgesehen.

Der Deckel umfasst eine Deckeloberfläche beziehungsweise eine Deckelplatte, die erfindungsgemäß eine weitgehend kreisrunde Scheibe bildet. Weiterhin weist der Deckel einen Deckelrand auf, der besonders bevorzugt sich von der Deckelplatte weg erstreckt. Auf diese Weise wird ein Zylindermantel unterhalb der Platte das heißt beim Gebrauch des Deckels in Richtung des Gehäuses erstreckender Deckelrand gebildet. Vorzugsweise umfasst der Deckelrand auch die radiale Oberfläche der Deckelplatte.

Das erste Rastelement ist gemäß einer Ausgestaltung als Wulst, Rastnase oder Vorsprung ausgeführt. Das erste Rastelement kann dabei als ein zusammenhängender kreisrunder Wulst auf der Innenseite des Deckelrandes ausgeführt sein. In einer weiteren Ausgestaltung ist vorgesehen, dass das erste Rastelement eine Anzahl von Wulsten oder Vorsprüngen aufweist, die innenseitig am Deckelrand angeordnet sind.

Der Deckel weist eine Anzahl von den Deckel durchgreifenden Ausnehmungen auf, die dem Benutzer ermöglichen, ausreichend Luft ein- und auszuatmen und insbesondere durch einen unter dem Deckel angeordneten Filter zu leiten. Erfindungsgemäß weist die Deckelplatte zwei oder mehr Ausnehmungen auf. Besonders bevorzugt ist vorgesehen, dass etwa 30 % bis etwa 90 % der Deckeloberfläche mit Ausnehmungen durchsetzt sind.

Besonders bevorzugt ist eine Ausgestaltung vorgesehen, bei der Ausnehmungen in einem Winkel von etwa 30° bis etwa 60°, bevorzugt etwa 45°, zu einer Normalen auf einer Deckeloberfläche den Deckel durchgreifen. Die Ausnehmungen oder Löcher gehen somit schräg beziehungsweise diagonal durch den Deckel. Dies hat den Vorteil, dass ein direkter Durchgriff beziehungsweise ein Sichtbarwerden des unter dem Deckel liegenden Filters oder anderer Vorrichtungen durch die Ausnehmungen vermieden werden kann. Dennoch ist es dem Benutzer möglich, gut und sicher durch den Deckel zu atmen. Ein weiterer Vorteil an der beschriebenen Ausführungsform ist, dass ein für den Benutzer ansprechendes Design erreicht werden kann. Ein weiterer Vorteil der schräg oder diagonal durch den Deckel ausgeführten Ausnehmungen oder Bohrungen ist, dass, insbesondere wenn Personen sich dem Benutzer nähern, ein Anblasen Dritter vermieden wird. Die Luft kann durch diese Ausgestaltung vorteilhaft nach unten oder zur Seite abgelenkt werden. Auch kann Spritzwasser mit dieser Konstruktion zuverlässig abgeschirmt werden.

In einer weiteren Ausführungsform ist vorgesehen, dass zumindest ein Teil der Ausnehmungen Bohrungen sind. Im Sinne der Erfindung sind unter Bohrungen runde, kreisrunde, aber auch ovale Ausnehmungen zu verstehen, die vollständig durch den Deckel hindurchgehen beziehungsweise diesen durchgreifen. Die Bohrungen können orthogonal zur Deckeloberfläche oder in einem Winkel von etwa 30° bis etwa 60°, bevorzugt etwa 45°, zu einer Normalen auf der Deckeloberfläche den Deckel durchgreifen. In einer weiteren Ausgestaltung ist vorgesehen, dass die Ausnehmungen gitterförmig auf der Deckeloberfläche angeordnet sind. Insbesondere sind Bohrungen oder sonst wie gestaltete Ausnehmungen gitterförmig vorgesehen. Eine weitere Ausgestaltung sieht vor, dass die Ausnehmungen einen über ihre Länge veränderlichen Querschnitt beziehungsweise eine veränderliche Öffnungsweite aufweisen. Insbesondere weiten sich die Ausnehmungen in distaler oder proximaler Richtung auf. Besonders bevorzugt ist ebenfalls, dass zumindest ein Teil der Ausnehmungen Schlitze sind. In einer weiteren Ausgestaltung ist vorgesehen, dass alle Ausnehmungen als Schlitze ausgestaltet sind. Weiterhin vorteilhaft sind die Schlitze, wie oben erwähnt, beispielsweise diagonal oder schräg und/oder mit sich veränderlichem Querschnitt vorgesehen.

Gemäß einer weiteren Ausgestaltung ist vorgesehen, dass Lamellen zwischen den Ausnehmungen angeordnet sind. Die Ausnehmungen sind vorteilhafterweise als Schlitze ausgestaltet. Die Schlitze erstrecken sich gemäß einer Ausgestaltung über eine gesamte Breite des Deckels. Weiter bevorzugt sind die Schlitze parallel zueinander angeordnet. Die Lamellen können in einer weiteren Ausgestaltung durch die Vorsehung der Schlitze gebildet sein.

Erfindungsgemäß ist vorgesehen, dass der Deckelrand außenseitig zumindest ein teilweise umlaufendes zweites Rastelement umfasst. Das zweite Rastelement ist insbesondere ausgestaltet als vollständig oder teilweise umlaufende Wulst, Vorsprung oder anderweitige Ausprägung. Weiterhin vorteilhaft sind mehrere Ausprägungen vorgesehen, die zusammen das Rastelement bilden. Das zweite Rastelement auf der Außenseite des Deckelrandes ist insbesondere dafür vorgesehen, einen Schild aufzunehmen, insbesondere einen Schild, welcher einen Verschluss des Deckels beziehungsweise des Atemdurchgangs vereinfacht.

Insbesondere ist in einer Ausgestaltung vorgesehen, dass das mindestens eine zweite Rastelement am Deckel vorgesehen ist, ohne dass das mindestens eine erste Rastelement am Deckel vorgesehen ist. In einer weiteren Ausführungsform ist vorgesehen, dass das mindestens eine erste Rastelement ohne ein zweites oder weiteres Rastelement vorgesehen ist. In einer weiteren Ausführungsform ist vorgesehen, dass das mindestens eine erste Rastelement und das mindestens eine zweite Rastelement vorgesehen sind. Weiterhin sieht eine Ausführungsform vor, dass das mindestens eine erste Rastelement und/oder das mindestens eine zweite Rastelement und zumindest ein weiteres Rastelement vorgesehen sind. Das mindestens eine erste Rastelement und/oder das mindestens eine zweite Rastelement können somit unabhängig voneinander und je nach Anforderung durch die vorgesehenen, mit dem Deckel in Verbindung gebrachten Bauteile am Deckel angeordnet sein.

In einer weiteren Ausführungsform ist vorgesehen, dass der Schild auf das Deckel aufklemmbar ist. Insbesondere ist gemäß einer Ausgestaltung vorgesehen, dass der Schild kein Rastelement aufweist. Weiterhin ist gemäß einer Ausführungsform vorgesehen, dass der Schild mindestens einen zumindest teilweise umlaufenden Wulst insbesondere zur Klemmung auf dem Deckel umfasst. Weiterhin sieht eine Variante des Schildes vor, dass dieser mit dem Deckel mittels einer kraftschlüssigen und/oder formschlüssigen Verbindung verbindbar ist.

Weiterhin wird ein Gehäuse zur Befestigung an dem oben beschriebenen Deckel vorgeschlagen, wobei das Gehäuse eine Rastaufnahme zur Aufnahme eines ersten Rastelementes des Deckels aufweist.

Gemäß einer Ausgestaltung ist vorgesehen, dass der Deckel auf dem Gehäuse aufklemmbar ist.

Vorteilhafterweise ist vorgesehen, dass der Deckel mittels der beschriebenen ersten Rastverbindung, die insbesondere aus dem ersten Rastelement des Deckels und der Rastaufnahme des Gehäuses besteht, oder diese Einzelteile zumindest umfasst, auf dem Gehäuse befestigbar ist. Wie bereits erwähnt, ist vorteilhafterweise vorgesehen, dass die erste Rastverbindung derart ausgestaltet ist, dass der Deckel drehbar auf dem Gehäuse angeordnet ist. Weiterhin ist durch diese Ausgestaltung eine kompakte Bauweise erreicht.

Weiterhin wird ein Schild zur Befestigung an dem Deckel vorgeschlagen, wobei der Schild eine zumindest teilweise umlaufende innenseitige Rastaufnahme für ein zweites Rastelement des Deckels umfasst. Der Deckel ist vorzugsweise mittels einer zweiten Rastverbindung, die zumindest das zweite Rastelement des Deckels und die Rastaufnahme des Schildes umfasst, an dem Schild befestigbar. Der Schild ist vorzugsweise derart ausgestaltet, dass dieser eine Atemöffnung aufweist, die im Wesentlichen so groß ist wie der Durchmesser des Deckels. In einer weiteren Ausgestaltung ist vorgesehen, dass das Atemloch des Schildes etwa 1 % bis etwa 5 % kleiner ist als der Durchmesser des Deckels. Besonders bevorzugt ist vorgesehen, dass das Atemloch zentrisch über dem Deckel anordbar ist. Weiterhin bevorzugt ist in einer Ausgestaltung vorgesehen, dass der Schild um das Atemloch herum eine Einbuchtung beziehungsweise eine trichterförmige Ausgestaltung aufweist. Die trichterförmige Ausgestaltung kann sowohl kreisrund als auch oval ausgestaltet sein, um insbesondere das Auflegen eines Fingers zu erleichtern. Die erfindungsgemäße trichterförmige Ausgestaltung ist vorteilhaft, weil dadurch der Finger auf das Atemloch geführt wird bei einem gewollten Verschluss desselben. Der Benutzer legt den Finger auf das Atemloch, um die ausgeatmete Luft durch eine Stimmprothese zu leiten. Durch die vorgeschlagene Ausgestaltung ist eine leichtere Abdichtung, insbesondere durch geringeren Druck auf den Schild beziehungsweise den Feuchte-Wärme-Tauscher, notwendig um einen dichtenden Verschluss zu erzielen.

In einer besonders bevorzugten Ausgestaltung ist vorgesehen, dass der Schild und/oder der Deckel aus einem gummielastischen Kunststoff oder einem elastischen Material hergestellt ist. Beispielsweise ist vorgesehen, dass der Schild und/oder der Deckel eine Material umfasst, ausgewählt aus einer Gruppe zumindest umfassend ein Metall, Polypropylen und/oder Polyvinylchlorid. In einer Ausgestaltung ist vorgesehen, dass der Schild mittels eines generativen Fertigungsverfahren , beispielsweise mittels eines 3D-Druckers hergestellt ist.

Gemäß einer Ausgestaltung ist vorgesehen, dass die Rastaufnahme des Schilds mindestens eine Nut aufweist, die vorzugsweise zumindest teilweise, bevorzugt vollständig kreisrund umlaufend ausgestaltet ist. Insbesondere ist die zumindest eine Nut in einem Längsschnitt, das heißt einem Schnitt von proximal nach lateral, C-förmig oder L-förmig ausgebildet. Weiter bevorzugt ist vorgesehen, dass die Nut einmal oder mehrfach unterbrochen ist, das heißt, dass die Rastaufnahme eine Anzahl von Nuten aufweist, die nebeneinander angeordnet sind, oder eine Nut aufweist, die nicht vollständig oder vollständig umlaufend ausgestaltet ist. Vorteilhafterweise ist gemäß einer Ausgestaltung vorgesehen, dass das zweite Rastelement des Deckels korrespondierend zu der Rastaufnahme, insbesondere der Nut, des Schilds ausgestaltet ist. Das heißt, dass das zweite Rastelement die gleiche Anzahl von Ausprägungen aufweist, die in die Nut oder Nute eingreifen können, wie Nute in der Rastaufnahme vorgesehen sind. Weiterhin bevorzugt ist vorgesehen, dass die Nute der Rastaufnahme und die Ausprägungen des zweiten Rastelements aufeinander abgestimmt sind, insbesondere im Wesentlichen passgenau ausgestaltet sind. Vorteil an der beschriebenen Ausgestaltung mit einer unterbrochenen Nut ist, dass der Schild verdrehsicher auf dem Deckel aufsitzt.

Ein weiterer Aspekt des vorgeschlagenen Feuchte-Wärme-Tauschers ist ein Bausatz umfassend zumindest eine Anzahl von Gehäusen, wobei die Gehäuse eine Rastaufnahme zur Aufnahme eines ersten Rastelements eines Deckels aufweisen, wie oben beschrieben, eine Anzahl von Deckeln, wie oben beschrieben, und eine Anzahl von Schilden, wie oben beschrieben. Vorzugsweise ist der Bausatz für einen Feuchte-Wärme-Tauscher mit unterschiedlichen Gehäusen und/oder unterschiedlichen Deckeln und/oder unterschiedlichen Schilden ausgestattet. Der Vorteil an dem vorgeschlagenen Bausatz ist, dass die medizinische Versorgung, beziehungsweise die Versorgung mit einem Feuchte-Wärme-Tauscher an den Benutzer individuell angepasst werden kann. Der Bausatz zeichnet sich insbesondere dadurch aus, dass in einer Ausgestaltung vorgesehen ist, dass die Gehäuse Einwegartikel sind. Das hat den Vorteil, dass nicht der komplette Feuchte-Wärme-Tauscher ausgewechselt beziehungsweise weggeschmissen werden muss, sondern einzelne Teile nach Reinigung wieder verwendet werden können und nur das Gehäuse, in einer weiteren Ausgestaltung nur der Filter, der im Gehäuse angeordnet ist, ausgetauscht wird. In einer weiteren Ausgestaltung ist vorgesehen, dass alle Teile des Bausatzes mehrfach verwendet werden können. In einer weiteren Variante ist vorgesehen, dass die Schilde eine Anzahl von unterschiedlichen Farben oder Außenformen aufweisen. Beispielsweise können die Außenformen bei einer Draufsicht im Wesentlichen rund oder im Wesentlichen oval ausgestaltet sein. Auch sind unterschiedliche Materialien oder Materialkombinationen für die Schilde in weiteren Ausgestaltungen vorgesehen. So sind beispielsweise unterschiedliche Elastizitäten für die verschiedenen Zwecke beziehungsweise Bedürfnisse des Benutzers vorgesehen. In einer weiteren Ausführungsform ist vorgesehen, dass die Schilde unterschiedliche Atemöffnungen und/oder Trichterformen aufweisen.

Der Bausatz ist in einer Ausgestaltung derart ausgeführt, dass die Deckel eine Anzahl von unterschiedlich ausgestalteten Ausnehmungen aufweisen. So sind Ausnehmungen vorgesehen, die im Querschnitt rund oder oval sind. Weitere Ausgestaltungen sehen vor, dass eine weitere Anzahl von Ausnehmungen gerade durchlaufen und/oder diagonal oder schräg den Deckel durchgreifen.

In einer weiteren Ausgestaltung ist vorgesehen, dass die Deckel unterschiedliche Farben aufweisen. Der Bausatz hat den weiteren Vorteil, dass nicht nur medizinisch angepasste Leistungen auf den Benutzer zugeschnitten werden können sondern auch, dass das Design entsprechend dem Geschmack der Benutzer ausgestaltet sein kann.

In einer weiteren Ausgestaltung ist vorgesehen, dass die Deckel auf den Gehäusen aufklemmbar sind.

In einer weiteren Ausführungsform ist vorgesehen, dass die Deckel mittels einer ersten Rastverbindung auf dem Gehäuse befestigbar sind.

In einer weiteren Variante ist vorgesehen, dass die Deckel drehbar auf dem Gehäuse sind.

Weiterhin wird ein Gehäuse eines Feuchte-Wärme-Tauschers für Tracheotomierte und Laryngektomierte umfassend einen Gehäusezylindermantel vorgeschlagen. Das Gehäuse weist weiterhin eine Filterrückhaltevorrichtung auf, wobei die Filterrückhaltevorrichtung zumindest einen Steg umfasst, der einer Grundfläche des Gehäuses zugeordnet ist. Der Gehäuse zylindermantel weist zumindest drei Einkerbungen, die insbesondere als Filmscharnier ausgestaltet sind, auf, die ein definiertes Einbeulen des Gehäusezylindermantels ermöglichen.

Vorteilhafterweise kann das Gehäuse in oder auf ein Tracheostomapflaster, ein Tracheostomabutton und/oder eine Tracheostomakanüle eingesetzt werden. Das Gehäuse ist bevorzugt zylindrisch ausgestaltet, wobei eine Grundfläche des Gehäuses durch die Filterrückhaltevorrichtung gebildet wird. Die Filterrückhaltevorrichtung weist einen oder mehrere Stege auf, insbesondere ist vorgesehen, dass die Stege miteinander im Verbund stehen. Der Gehäusezylindermantel weist weiterhin zumindest drei Einkerbungen auf, die in einer bevorzugten Ausgestaltung Filmscharniere sind. In einer weiteren Ausgestaltung ist vorgesehen, dass die Einkerbungen als Sollbruchstellen ausgestaltet sind. Die Einkerbungen sind vorzugsweise derart über den Gehäusezylindermantel verteilt, dass durch Druck, insbesondere Fingerdruck auf den Gehäusezylindermantel dieser eingebeult wird. Unter einer Einbeulung ist im Sinne der Erfindung zu verstehen, dass die durch die Einkerbungen definierten Sektionen des Gehäusezylindermantels zueinander bewegt und insbesondere radial eingedrückt werden.

Der Vorteil an dem beschriebenen Gehäuse ist, dass, sofern ein Deckel oder ein weiteres Bauteil auf dem Gehäuse angeordnet ist, dieses leicht aus der Rastverbindung gelöst werden kann, indem ein Druck, insbesondere Fingerdruck radial auf den Gehäusezylindermantel ausgeübt wird. Sodann beult sich der Gehäusezylindermantel definiert ein, so dass dieser aus einer Rastverbindung mit einem weiteren Bauteil, insbesondere einem Deckel, gelöst wird.

In einer bevorzugten Ausgestaltung ist vorgesehen, dass die Einkerbungen in einem Winkel von etwa 15° bis etwa 75°, weiter bevorzugt etwa 50° bis etwa 70°, weiter bevorzugt etwa 60° zueinander angeordnet sind. Die Anordnung der Kerben und insbesondere auch die Anzahl der Kerben bestimmen den Grad der Einbeulung und den Widerstand, den der Gehäusezylindermantel bei Druck auf selbigen ausübt.

Wird im Rahmen der Erfindung der Begriff "etwa" verwendet, so ist darunter ein Toleranzbereich zu verstehen, den der Fachmann auf diesem Gebiet für üblich erachtet, insbesondere ist ein Toleranzbereich von +/- 20%, bevorzugt +/- 10% vorgesehen. Auch der Begriff "im Wesentlichen" gibt einen Toleranzbereich an, der für den Fachmann unter wirtschaftlichen und technischen Gesichtspunkten zu vertreten ist, so dass das einsprechende Merkmal noch als solches zu erkennen ist.

In einer weiteren Ausgestaltung ist vorgesehen, dass die Einkerbungen auf dem Gehäusezylindermantel über einen Winkel von etwa 90° bis etwa 180°, bevorzugt etwa 100° bis etwa 160°, weiter bevorzugt etwa 120° verteilt angeordnet sind.

In einer besonders bevorzugten Ausgestaltung ist vorgesehen, dass die Kerben auf der Innenseite des Gehäusezylindermantels angeordnet sind. In einer weiteren Ausgestaltung ist vorgesehen, dass zumindest eine Einkerbung auf der Außenseite des Gehäusezylindermantels angeordnet ist. Die Einkerbungen sind insbesondere materielle Verdünnungen beziehungsweise Querschnittsverjüngungen des Gehäusezylindermantels, die ein- oder beidseitig am Gehäusezylindermantel angeordnet sind. Durch die verschiedenen Ausgestaltungen der Einkerbungen, wie beispielsweise dreieckig im Querschnitt oder sanft übergehend beziehungsweise gleichmäßig übergehend zur Materialdicke des restlichen Gehäusezylindermantels, können verschiedene Eigenschaften insbesondere der Beulung erstrebt sein. Durch das Einbeulen, beziehungsweise die radiale Kraftausübung auf den Zylindermantel, entstehen Zug- und Druckkräfte auf der Außen- und Innenseite des Gehäusezylindermantels. Diese Zug- und Druckkräfte können durch die Materialverdünnungen beziehungsweise Einkerbungen definiert respektive gezielt in Verformungen umgewandelt werden. Insbesondere ist vorgesehen, dass die Einbeulung leichter und über einen größeren Umfang erfolgt als bei einem gleichwertigen Druck auf den Gehäusezylindermantel ohne solche Einkerbungen. In einer Ausgestaltung, bei der die Einkerbungen als Sollbruchstellen ausgestaltet sind, soll insbesondere durch leichten Druck auf den Gehäusezylindermantel das definierte Brechen desselben herbeigeführt werden. Insbesondere sind die Einkerbungen derart ausgestaltet, dass Verletzungen durch scharfe Kanten und/oder ein unkontrolliertes Brechen, was beispielsweise durch einen zu hohen Kraftaufwand, der die Handhabung nicht mehr kontrollierbar machen würde, vermieden werden.

In einer weiteren Variante des Gehäuses ist vorgesehen, dass die Filterrückhaltevorrichtung einen Anschlag für den eingebeulten Gehäusezylindermantel umfasst. Der Anschlag ist insbesondere derart gestaltet, dass dieser gegenüberliegend der voraussichtlich größten Einbeulung angeordnet ist. Der Anschlag ist vorzugsweise derart gestaltet, dass der Gehäusezylindermantel nur soweit eingedrückt wird, dass entweder kein Bruch des Gehäusezylindermantels erfolgt, oder bei einem gewollten Bruch des Gehäusezylindermantels im Bereich der Einkerbungen diesen begrenzt, um beispielsweise vollständiges Brechen zu vermeiden.

Im Rahmen der Erfindung ist unter einer Sollbruchstelle beziehungsweise einem Bruch eine Bruchstelle, auch eine Weißbruchstelle in einem Kunststoffteil, zu verstehen. Weißbruch sind kleine Bereiche, deren Begrenzungsflächen mit einzelnen extrem verstreckten Materialsträngen überbrückt sind. Diese extrem verstreckten Materialstränge werden Crazes genannt und sind eine Vorschädigung des Materials.

Die Filtervorrichtung kann Teil eines Steges sein oder bevorzugt an dem Steg angeordnet. Insbesondere ist der Anschlag eine an dem Steg angeordnete Ausprägung oder durch einen Steg gebildet.

Vorzugsweise ist das Gehäuse aus einem Material gefertigt. In einer weiteren Ausgestaltung ist vorgesehen, dass das Gehäuse mehrere Materialien umfasst. Weiterhin bevorzugt ist das Gehäuse einteilig. In einer weiteren Ausgestaltung ist vorgesehen, dass das Gehäuse mehrteilig ausgestaltet ist. Besonders bevorzugt weist das Gehäuse ein Material ausgewählt aus einer Gruppe zumindest umfassend Polypropylen und/oder Polyvinylchlorid. In einer Ausgestaltung ist vorgesehen, dass das Gehäuse mittels eines generativen Fertigungsverfahren, beispielsweise mittels eines 3D-Druckers hergestellt ist.

Gemäß einer weiteren Ausführungsform ist vorgesehen, dass die Filterrückhaltevorrichtung eine Anzahl von Stegen aufweist. Die Anzahl von Stegen kann parallel, orthogonal oder in geometrischen Formen zueinander angeordnet sein. Insbesondere ist vorgesehen, dass die Stege radial angeordnet sind. Die Stege weisen Stegenden auf, die gemäß einer Ausgestaltung mit dem Gehäusezylindermantel und/oder weiteren Stegen verbunden sind. Gemäß einer besonders bevorzugten Ausgestaltung ist vorgesehen, dass Stegenden der Stege über einen Winkel über etwa 120° bis etwa 200° auf einem Innenumfang des Gehäusezylindermantels verteilt sind. Insbesondere ist vorgesehen, dass Stegenden der Stege über einen Winkel von etwa 180° auf einem Innenumfang des Gehäusezylindermantels verteilt sind. Implizit bedeutet dies, dass der restliche Zylindermantel frei von Stegenden ist und sich besonders bevorzugt hier anbietet, die Kerbungen für die Einbeulungen vorzusehen. So ist gemäß einer Ausgestaltung vorgesehen, dass das Gehäuse eine Anzahl von Stegen aufweist, wobei die Stegenden über einen Winkel von etwa 180° auf den Innenumfang des Gehäusezylindermantels verteilt sind und der Gehäusezylindermantel verteilt auf die andere Hälfte des Zylindermantels mindestens drei Einkerbungen aufweist, die entweder ebenfalls etwa über einen Winkel von 180°, bevorzugt jedoch in einem Winkel von etwa 120°, über den freibleibenden Teil des Gehäusezylindermantels verteilt sind.

In einer besonders bevorzugten Ausgestaltung ist vorgesehen, dass das Gehäuse eine Deckelaufnahme für einen Deckel aufweist. Der Deckel ist insbesondere ein Schutz für einen Filter, der einerseits das Herausfallen des Filters und andererseits den direkten Sichtkontakt und/oder die direkte Berührung des Filters verhindern soll. Vorzugsweise ist der Deckel mit Ausnehmungen ausgestaltet. Die Deckelaufnahme ist vorzugsweise ausgestaltet als ein zumindest teilweise umlaufender Wulst, der an der gegenüberliegend der Filterrückhaltevorrichtungen angeordneten Seite des Gehäusezylindermantels angeordnet ist. Die Deckelaufnahme, insbesondere der Wulst, kann Unterbrechungen aufweisen oder nur partiell auf dem Umfang des Gehäusezylindermantels verteilt sein. In einer weiteren Ausgestaltung ist vorgesehen, dass die Deckelaufnahme auf einem Innen- und/oder Außenumfang des Gehäusezylindermantels angeordnet ist. Besonders bevorzugt ist die Deckelaufnahme eine auf der Außenseite des Gehäusezylindermantels angeordnete Rastvorrichtung. Dadurch, dass die Deckelaufnahme auf der Außenseite des Gehäusezylindermantels angeordnet ist, kann bei einem Einbeulen des Gehäusezylindermantels durch radialen Druck auf selbigen die Rastvorrichtung der Deckelaufnahme aus einer Rastnut des Deckels herausgeführt und so der Deckel von dem Gehäuse gelöst werden.

Es versteht sich, dass in einer Ausgestaltung vorgesehen ist, dass der Deckel auf das Gehäuse aufgerastet wird. Insbesondere wird ein Verfahren zur Lösung eines Deckels von einem Gehäuse, wie oben beschrieben, vorgeschlagen, wobei eine Kraft senkrecht auf eine äußere Oberfläche eines Gehäusezylindermantels eingeleitet wird, insbesondere radial auf selbigen, so dass der Gehäusezylindermantel bedingt durch Einkerbungen definiert einbeult und der Deckel aus einer Rastverbindung gelöst wird.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf.
- Fig. 1: zeigt ein Gehäuse;
- Fig. 2: zeigt das Gehäuse in der Draufsicht;
- Fig. 3: zeigt das Gehäuse aus Fig. 2 in einer Schnittansicht;
- Fig. 4: zeigt einen Deckel mit Lamellen;
- Fig. 5: zeigt den Deckel in der Draufsicht;
- Fig. 6: zeigt eine Schnittansicht der Fig. 5;
- Fig. 7: zeigt eine weitere Version eines Deckels mit Bohrungen;
- Fig. 8: zeigt den Deckel aus Fig. 7 in einer Draufsicht;
- Fig. 9: zeigt den Deckel aus Fig. 8 in einer Schnittansicht;
- Fig. 10: zeigt einen Schild;
- Fig. 11: zeigt eine Variante des Schildes in kreisrunder Ausgestaltung;
- Fig. 12: zeigt eine Variante des Schildes in ovaler Ausgestaltung;
- Fig. 13: zeigt eine Schnittansicht eines Schildes;
- Fig. 14: zeigt einen Bausatz bestehend aus einer Anzahl von Gehäusen, einer Anzahl von Gehäusen, einer Anzahl von Deckeln und einer Anzahl von Schilden;
- Fig. 15: zeigt ein Verfahren zur Benutzung eines Feuchte-Wärme-Tauschers umfassend den Bausatz aus Fig. 14;
- Fig.16: zeigt eine weitere Ausführung des Deckels;
- Fig. 17: zeigt eine Schnittansicht des Deckels aus Fig. 16;
- Fig. 18: zeigt eine weitere Ausführung des Deckels; und
- Fig. 19: zeigt eine Schnittansicht des Deckels aus Fig. 18.

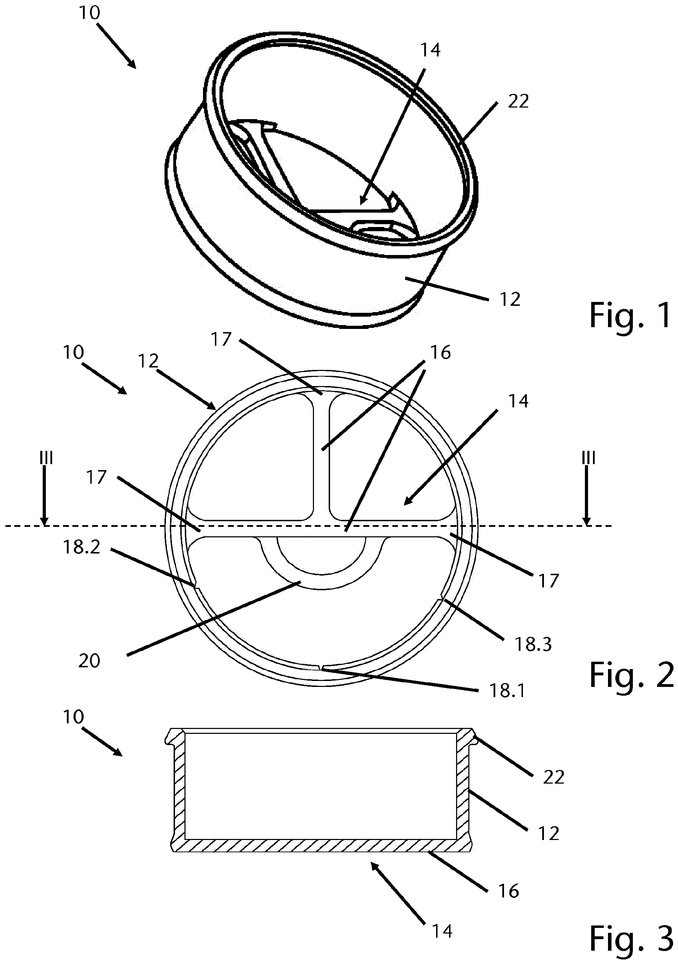

Fig. 1 zeigt ein Gehäuse 10 eines Feuchte-Wärme-Tauschers für tracheotomierte und laryngektomierte Patienten. Das Gehäuse 10 weist einen Gehäusezylindermantel 12 sowie eine Filterrückhaltevorrichtung 14 auf. In das Gehäuse 10 wird für den Gebrauch ein hier nicht dargestellter Filter, insbesondere aus einem offenporigen Schaumstoff eingesetzt und derart in eine Tracheostomavorrichtung wie beispielsweise ein Tracheostomapflaster oder eine Trachialkanüle eingesetzt, so dass die Filterrückhaltevorrichtung 14 proximal zum Tracheostoma sitzt. Die Filterrückhaltevorrichtung 14 verhindert ein versehentliches Einatmen des Filters. Der Fig. 1 ist weiterhin eine außen am Gehäusezylindermantel umlaufende Deckelaufnahme 22 zu entnehmen, die als Wulst an der distalen Seite ausgestaltet ist.

Fig. 2 zeigt das Gehäuse 10 in einer Draufsicht, wobei die Filterrückhaltevorrichtung 14 detailliert zu erkennen ist. Diese weist zwei zueinander angeordnete Stege 16 auf, deren Stegenden 17, die radial außenliegend sind, sind mit der Innenseite des Gehäusezylindermantels 12 verbunden, beziehungsweise gehen in diesen über. Es ist zu erkennen, dass an einem der Stege 16 ein Anschlag 20 angeordnet ist, der in dieser Ausgestaltung einen Kreisbogen bildet. Weiterhin ist der Fig. 2 zu entnehmen, dass der Gehäusezylindermantel 12 drei Einkerbungen 18 aufweist, die den Gehäusezylindermantel 12 definiert schwächen. Ein radialer Druck auf beispielsweise den Gehäusezylindermantel im Bereich der Kerbe 18.1 bewirkt, dass sich der Gehäusezylindermantel einbeulen kann. Die definierte Einbeulung wird durch die Kerben 18.2 und 18.3 in ihrem Umfang im Wesentlichen begrenzt. Weiterhin wird die Einbeulung durch den Anschlag 20 begrenzt, so dass keine ungewollten oder zu starken Verformungen beziehungsweise unkontrollierten Brüche geschehen.

Fig. 3 zeigt eine Schnittansicht der Fig. 2 im Schnitt III-III Dieser Ansicht ist ebenfalls der Gehäusezylindermantel 12 sowie die Filterrückhaltevorrichtung 14 mit dem Steg 16 zu entnehmen. Weiterhin ist zu erkennen, dass das Gehäuse 10 einen Wulst an der distalen Seite aufweist, der die Deckelaufnahme 22 bildet.

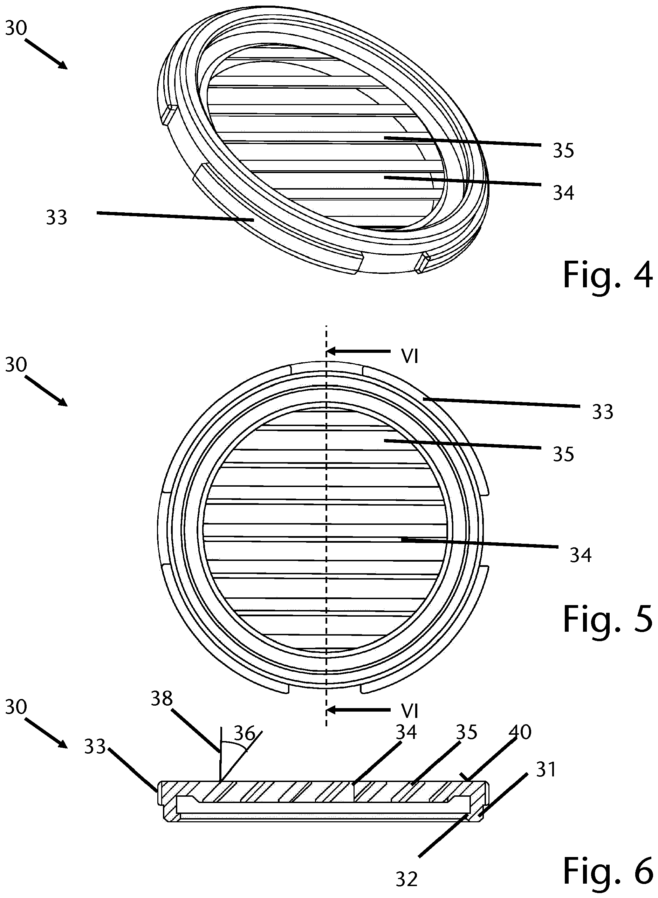

Fig. 4 zeigt einen Deckel 30 für das Gehäuse 10 eines Feuchte-Wärme-Tauschers. Der Deckel 30 weist eine Anzahl von Ausnehmungen 34 auf, die von Lamellen 35 begrenzt sind. Weiterhin ist der Fig. 4 ein Rastelement 33 zu entnehmen, das insbesondere zur Aufnahme eines Schildes vorgesehen ist.

Fig. 5 zeigt den Deckel 30 aus Fig. 4 in einer Draufsicht. Es ist zu erkennen, dass die Lamellen 35 abgeschrägt verlaufen und die direkte Durchsicht durch die Ausnehmungen 34 relativ schmal ist, obwohl aus Fig. 6 hervor geht, dass die Ausnehmungen ausreichend groß sind, jedoch schräg durch den Deckel durchlaufen.

Wie weiterhin aus Fig. 6, der Schnittansicht der Fig. 5 durch den Schnitt VI-VI zu entnehmen ist, durchgreifen die Ausnehmungen 34 in einem Winkel 36 von etwa 45° zu einer Normalen 38 auf eine Deckeloberfläche 40 des Deckels 30. Weiterhin ist das teilweise umlaufende zweite Rastelement 33 den Fig. 4 bis 6 zu entnehmen, das den Deckelrand 31 außenseitig umläuft. Wie beispielsweise aus Fig. 5 zu entnehmen ist, umläuft das Rastelement 33 den Deckelrand 31 nicht vollständig, sondern weist Unterbrechungen auf. Weiterhin ist der Fig. 6 zu entnehmen, dass der Deckel 30 einen Deckelrand 31 umfasst, der innenseitig ein kreisrundumlaufendes erstes Rastelement 32 aufweist. Dieses Rastelement 32 wirkt zusammen mit der oben beschriebenen Deckelaufnahme 22 des Gehäuses als eine erste Rastverbindung.

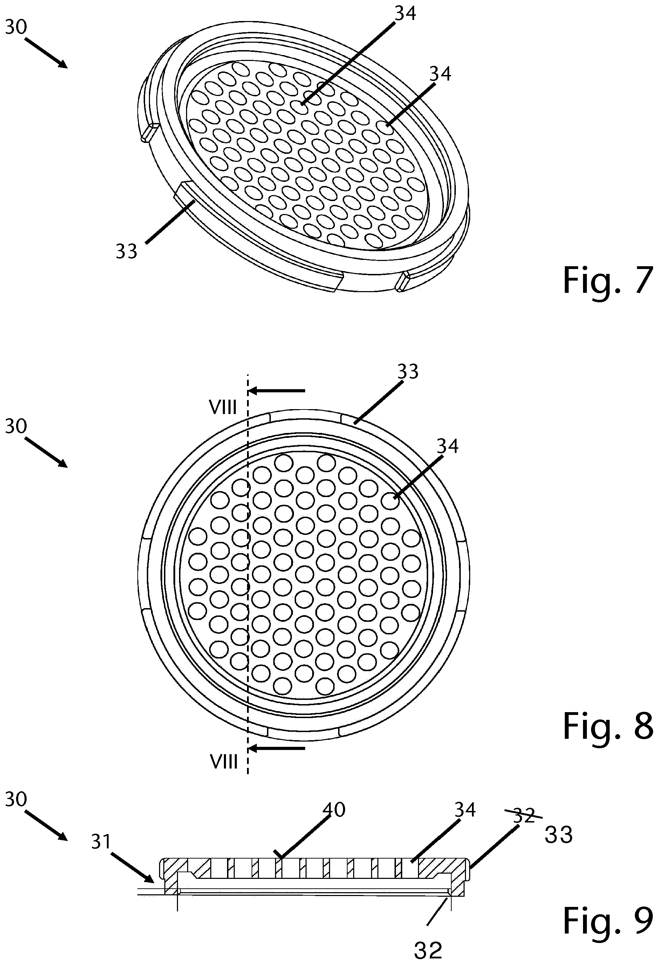

Die Fig. 7 bis 9 zeigen in eine weitere Ausgestaltung des Deckels 30, bei der die Ausnehmungen 34 als Bohrungen ausgestaltet sind. Die Bohrungen gehen in der gezeigten Ausgestaltung senkrecht zur Oberfläche 40 des Deckels 30 durch diesen hindurch.

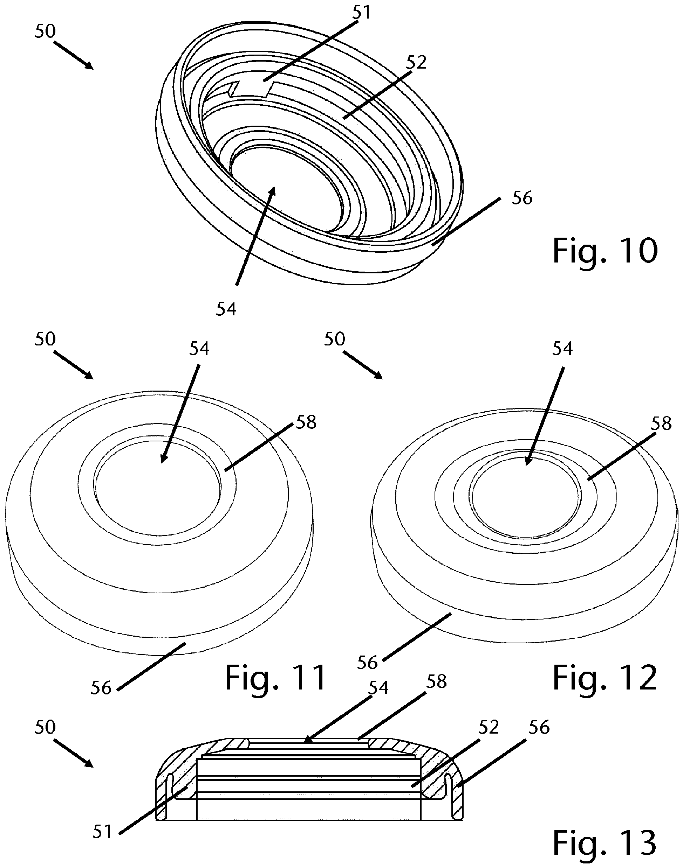

Fig. 10 zeigt einen Schild 50 zur Befestigung auf einen Deckel 30. Der Schild 50 weist eine zumindest teilweise umlaufende innenseitige Rastaufnahme 52 auf, die mit dem zweiten Rastelement 33 des Deckels 30 eine zweite Rastverbindung bildet. Der Schild 50 weist weiterhin eine Atemöffnung 54 auf, die insbesondere derart ausgestaltet ist, dass bei Aufsetzen oder nach dem Aufsetzen auf den Deckel 30 die Ausnehmungen komplett frei liegen. Somit wird ein ungehindertes Atmen durch den zusammengebauten Feuchte-Wärme-Tauscher ermöglicht. Der Schild 50 weist einen das Gehäuse 10 und den Deckel 30 umgreifenden Rand 56 auf, der insbesondere dafür sorgt, dass Gehäuse und Deckel und eventuelle Aufbauten des Tracheostomapflasters, der Trachealkanüle oder des Tracheostomabuttons nicht zu sehen sind. Auf diese Weise wird der ästhetische Anspruch eines Benutzers befriedigt, da der beschriebene zusammengebaute Feuchte-Wärme-Tauscher auch eine Schmuckfunktion erfüllt.

Fig. 11 zeigt eine Variante des Schildes 50, wobei der Schild 50 eine runde insbesondere etwa kreisrunde Ausgestaltung insbesondere im Querschnitt aufweist.

Fig. 12 zeigt eine weitere Ausgestaltung des Schildes 50, wobei dieses einen ovalen Querschnitt aufweist. Insbesondere ist auch die Atemöffnung 54 oval geformt.

Den Fig. 11 und 12 ist weiterhin zu entnehmen, dass die erfindungsgemäße Atemöffnung 54 trichterförmig ausgestaltet ist. Diese Trichterform erlaubt insbesondere bei einem gewollten Verschließen durch den Benutzer, eine angenehme Lage des Fingers auf dem zusammengebauten Feuchte-Wärme-Tauscher.

Fig. 13 zeigt eine Schnittansicht eines Schilds 50. Der Schnitt ist ausgeführt durch die Unterbrechungen 51 der Nut der Rastaufnahme 52, die aus Fig. 10 zu entnehmen sind. Aus dieser ist auch die Atemöffnung 54 sowie die Rastaufnahme 52 zu erkennen.

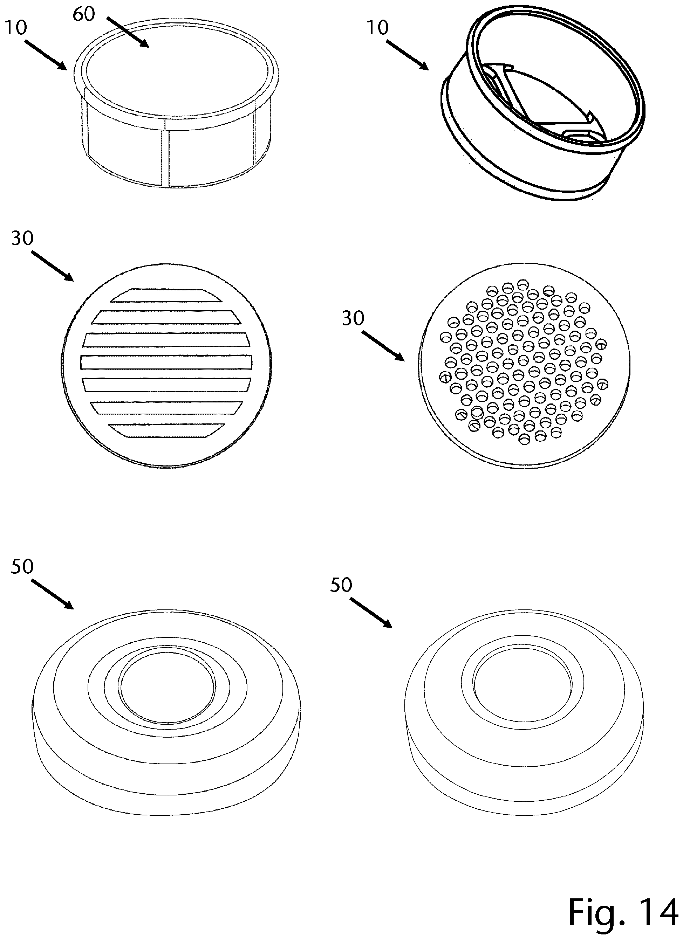

Fig. 14 zeigt einen Bausatz für einen Feuchte-Wärme-Tauscher. Dieser weist eine Anzahl von Gehäusen 10 und eine Anzahl von Deckeln 30 sowie eine Anzahl von Schilden 50 auf. In dem gezeigten Beispiel sind jeweils zwei Varianten der Gehäuse 10, der Deckel 30 und der Schilde 50 zu sehen. Ein Benutzer beziehungsweise ein medizinischer Betreuer, wie beispielsweise ein Arzt des Benutzers kann die passenden Komponenten für den Feuchte-Wärme-Tauscher auswählen. Hierbei spielen beispielsweise die Größe des Gehäuses beziehungsweise der Gehäuseaufnahme bei einem Tracheostomapflaster oder ähnliches sowie die gewollte Funktionalität als auch das gewünschte Design eine Rolle. Vorzugsweise ist vorgesehen, dass das Gehäuse als Einmalgehäuse ausgestaltet ist. Dieses weist einzeln verpackt oder direkt mitgeliefert einen Filter 60 auf, der in das Gehäuse eingesetzt wird. Nach der Benutzung beziehungsweise nach einer gewissen Dauer der Benutzung wird das Gehäuse samt Filter oder nur der Filter ausgetauscht. Weiterhin kann der Benutzer nach eigenem Ermessen einen Deckel aussuchen, der einerseits die direkte Sicht auf den Filter verhindert als auch ein Herausfallen des Filters in distale Richtung vermeidet. Weiterhin hat der Deckel eine Schutzfunktion insofern, dass gröbere Partikel, Flusen oder Anderweitiges und insbesondere auch bei einem Verschließen des Feuchte-Wärme-Tauschers der Finger des Benutzers nicht in direkten Kontakt mit dem Filter 60 kommt. Der Benutzer kann weiterhin aus einer Anzahl von Schilden 50 wählen, die insbesondere einem ästhetischen Anspruch genügen sollen. Weiterhin haben die Schilde auch den technischen Zweck, dass ein Verschließen des Feuchte-Wärme-Tauschers beispielsweise mit dem Finger komfortabel sein soll. Hierzu ist vorgesehen, dass einerseits der Schild 50 aus einem gummielastischen Material gefertigt ist und andererseits die Atemöffnung 54 trichterförmig ausgestaltet wird.

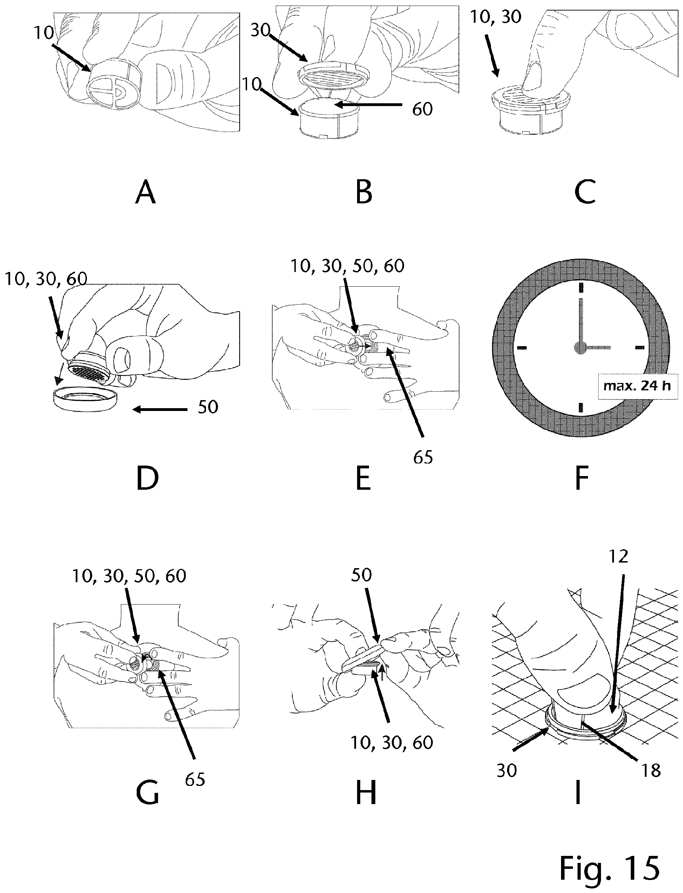

Fig. 15 zeigt die Verwendung des beschriebenen Feuchte-Wärme-Tauschers beziehungsweise dessen Bausatzes. Piktogramm A zeigt, wie ein Benutzer das Gehäuse 10 auf einen festen Untergrund legt. Sollte in das Gehäuse 10 noch kein Filter eingesetzt worden sein, so wird der Filter 60 nun eingesetzt und im folgenden Schritt, wie Piktogramm B zeigt, der Deckel 30 auf das Gehäuse 10 aufgesetzt und eingerastet. Piktogramm C zeigt, dass die Einrastung leicht mittels Fingerdruck erfolgen kann. Das folgende Piktogramm D zeigt, dass das mit dem Deckel 30 versehene Gehäuse 10 in einen Schild 50 eingesetzt wird. Auch hierzu wird vorgeschlagen, den Schild 50 auf eine feste Unterlage zu legen und das Gehäuse 10 mitsamt dem Deckel 30 in den Schild 50 einzudrücken. Wie in dem Piktogramm D klar zu erkennen ist, spielt hier der Deckel 30 eine zentrale Rolle. Dieser greift sowohl das Gehäuse 10 als auch den Schild 50. Gemäß einer bevorzugten Ausgestaltung ist es somit nicht möglich, das Gehäuse 10 ohne den Deckel 30 mit dem Schild 50 zu verbinden. Der Deckel 30 hat somit eine Kupplungsfunktion.

Piktogramm E zeigt, dass der fertig zusammengebaute Feuchte-Wärme-Tauscher 10, 30 und 50 in ein Tracheostomapflaster 65 eingesetzt wird. Piktogramm F soll verdeutlichen, dass der Feuchte-Wärme-Tauscher für einen gewissen Zeitraum getragen werden kann, wobei eine Höchsttragezeit von etwa 24 Stunden empfohlen wird. Je nach Verschmutzung oder Sekretbildung kann ein früherer Austausch oder gegebenenfalls auch ein späterer Austausch erfolgen. Nach dieser Zeit wird, wie in Piktogramm G verdeutlicht, der Feuchte-Wärme-Tauscher wieder entfernt und der Schild 50 von dem Deckel 30 gelöst. Nun wird mit leichtem Fingerdruck auf den Gehäusezylindermantel 12 im Bereich der Einkerbungen 18 das Gehäuse 10, insbesondere der Gehäusezylindermantel 12, eingebeult, bis sich der Deckel 30 von dem Gehäuse 10 löst.

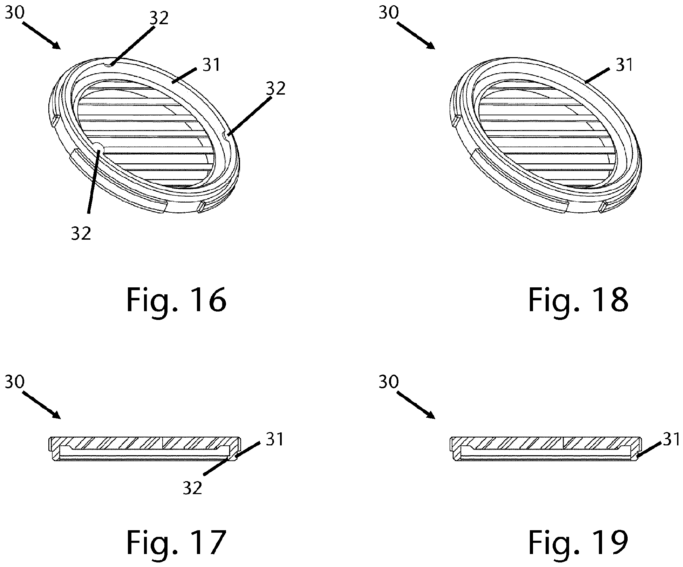

Fig. 16 zeigt eine weitere Ausgestaltung des Deckels 30 mit drei Rastelementen 32, die auf einem Innenumfang des Deckelrandes 31 angeordnet und als Rastnasen ausgebildet sind. Um den Aufbau möglichst kompakt zu gestalten, sind die Rastelemente 32 randnah, das heißt möglichst proximal am Deckelrand angeordnet. Die Rastelemente 32 ragen etwa 0,4 mm bis etwa 0,8 mm radial nach innen. Fig. 17 ist eine Schnittansicht des Deckels 30 aus Fig. 16.

Fig. 18 zeigt eine weitere Ausgestaltung des Deckels 30 ohne Rastelemente. Diese Ausgestaltung erlaubt ein Aufklemmen des Deckels 30 auf ein Gehäuse. Fig. 19 ist eine Schnittansicht des Deckels 30 aus Fig. 18.

## Patentansprüche

1. Deckel (30) mit einem Schild (50) zur Befestigung an dem Deckel (30) für ein Gehäuse (10) eines Feuchte-Wärme-Tauschers für Laryngektomierte und Tracheotomierte umfassend eine als weitgehend kreisrunde Scheibe ausgebildete Deckelplatte, einen Deckelrand (31) und eine Anzahl von den Deckel (30) durchgreifende Ausnehmungen (34), wobei die Deckelplatte zwei oder mehr Ausnehmungen aufweist, wobei innenseitig am Deckelrand (31) zumindest ein teilweise kreisrund umlaufendes erstes Rastelement (32) angeordnet ist, wobei der Deckelrand (31) außenseitig zumindest ein teilweise umlaufendes zweites Rastelement (33) umfasst, wobei der Schild eine Atemöffnung (54) aufweist, wobei der Schild (50) um die Atemöffnung (54) eine trichterförmige Ausgestaltung aufweist, um einen Finger auf das Atemloch bei einem gewollten Verschluss desselben zu führen, wobei der Schild (50) eine zumindest teilweise umlaufende innenseitige Rastaufnahme (52) für das zweite Rastelement (33) des Deckels (30) umfasst.

2. Deckel (30) mit Schild (50) nach Anspruch 1, **dadurch gekennzeichnet, dass** der Deckelrand (31) klemmbar auf einem Gehäuse (10) befestigbar ist.

3. Deckel (30) mit Schild (50) nach einem oder mehreren der vorhergehenden Ansprüche, **dadurch gekennzeichnet, dass** der Deckelrand (31) Klemmelemente aufweist.

4. Deckel (30) mit Schild (50) nach Anspruch einem oder mehreren der vorhergehenden Ansprüche, **dadurch gekennzeichnet, dass** die Rastelemente (32) am Innenumfang des Deckelrandes (31) umlaufend verteilt sind.

5. Deckel (30) mit Schild (50) nach einem oder mehreren der vorhergehenden Ansprüche, **dadurch gekennzeichnet, dass** Ausnehmungen (34) in einem Winkel (36) von etwa 30° bis etwa 60° zu einer Normalen (38) auf einer Deckeloberfläche (40) den Deckel (30) durchgreifen.

6. Deckel (30) mit Schild (50) nach einem oder mehreren der vorhergehenden Ansprüche, **dadurch gekennzeichnet, dass** zumindest ein Teil der Ausnehmungen (34) Bohrungen sind.

7. Deckel (30) mit Schild (50) nach einem oder mehreren der vorhergehenden Ansprüche, **dadurch gekennzeichnet, dass** zumindest ein Teil der Ausnehmungen (34) Schlitze sind.

8. Deckel (30) mit Schild (50) nach Anspruch 7, **dadurch gekennzeichnet, dass** Lamellen (35) zwischen den Schlitzen angeordnet sind.

9. Deckel (30) mit Schild (50) nach Anspruch 1, **dadurch gekennzeichnet, dass** der Deckel (30) mittels einer zweiten Rastverbindung an dem Schild (50) befestigbar ist.

10. Bausatz (10, 30, 50) umfassend zumindest eine Anzahl von Gehäusen (10), wobei die Gehäuse (10) eine Rastaufnahme (22) zur Aufnahme eines ersten Rastelements (32) eines Deckels (30) nach einem oder mehreren der Ansprüche 1 bis 9 aufweisen, und eine Anzahl von Deckel (30) mit Schild (50) nach einem der Ansprüche 1 bis 9.

11. Bausatz (10, 30, 50) nach Anspruch 10, **dadurch gekennzeichnet, dass** die Gehäuse (10) Einwegartikel sind.

12. Bausatz (10, 30, 50) nach einem der Ansprüche 10 oder 11, **dadurch gekennzeichnet, dass** die Schilde (50) eine Anzahl von unterschiedlichen Farben und/oder Außenformen aufweisen.

13. Bausatz (10, 30, 50) nach einem oder mehreren der Ansprüche 10 bis 12, **dadurch gekennzeichnet, dass** die Deckel (30) eine Anzahl von unterschiedlich ausgestalteten Ausnehmungen (34) aufweisen.

14. Bausatz (10, 30, 50) nach einem oder mehreren der Ansprüche 11 bis 13, **dadurch gekennzeichnet, dass** die Deckel (30) unterschiedliche Farben aufweisen.

15. Bausatz (10, 30, 50) nach einem oder mehreren der Ansprüche 10 bis 14, **dadurch gekennzeichnet, dass** die Deckel (30) auf den Gehäusen (10) aufklemmbar sind.

16. Bausatz (10, 30, 50) nach einem oder mehreren der Ansprüche 10 bis 15, **dadurch gekennzeichnet, dass** die Deckel (30) mittels einer ersten Rastverbindung auf den Gehäusen (10) befestigbar sind.

17. Bausatz (10, 30, 50) nach einem oder mehreren der Ansprüche 10 bis 16, **dadurch gekennzeichnet, dass** die Deckel (30) drehbar auf dem Gehäuse (10) sind.

## Claims

1. Cover (30) having a shield (50) for being attached to the cover (30) for a housing (10) of a moisture-heat exchanger for laryngectomized and tracheostomized patients, comprising a cover plate being shaped as a substantially circular plate, a cover edge (31) and a number of recesses (34) passing through the cover (30), wherein the cover plate has two or more recesses, wherein at least one partially circular, encircling first latching element (32) is arranged on the inner surface of the cover edge (31), wherein the cover edge (31) comprises an at least one partially encircling second latching element (33) on the outside, wherein the shield has a breathing opening (54), wherein the shield (50) has a funnel-shaped form around the breathing opening (54) for guiding a finger onto the breathing hole for closing it, wherein the shield (50) comprises an at least partially encircling inner latching element receiver (52) for the second latching element (33) of the cover (30).

2. Cover (30) with shield (50) according to claim 1, **characterized in that** the cover edge (31) is attached in a clamping manner to a housing (10).

3. Cover (30) with shield (50) according to one or more of the preceding claims, **characterized in that** the cover edge (31) has clamping elements.

4. Cover (30) with shield (50) according to one or more of the preceding claims, **characterized in that** the latching elements (32) are distributed over the circumference of the inner surface of the cover edge (31).

5. Cover (30) with shield (50) according to one or more of the preceding claims, **characterized in that** recesses (34) pass through the cover (30) at an angle (36) of approximately 30° to approximately 60° to a normal (38) of a plane of a cover surface (40).

6. Cover (30) with shield (50) according to one or more of the preceding claims, **characterized in that** at least a portion of the recesses (34) are bore-holes.

7. Cover (30) with shield (50) according to one or more of the preceding claims, **characterized in that** at least a portion of the recesses (34) are slots.

8. Cover (30) with shield (50) according to claim 7, **characterized in that** fins (35) are disposed between the slots.

9. Cover (30) with shield (50) according to claim 1, **characterized in that** the cover (30) can be attached to the shield (50) by means of a second latching connection.

10. Kit (10, 30, 50) comprising at least a number of housings (10), wherein the housings (10) have a latching recess (22) for receiving a first latching element (32) of a cover (30) according to one or more of the claims 1 to 9, and a number of covers (30) with shields (50) according to one of the claims 1 to 9.

11. Kit (10, 30, 50) according to claim 10, **characterized in that** the housings (10) are disposable.

12. Kit (10, 30, 50) according to one of the claims 10 or 11, **characterized in that** the shields (50) have a number of different colors and/or shapes.

13. Kit (10, 30, 50) according to one or more of the claims 10 to 12, **characterized in that** the covers (30) have a number of differently shaped recesses (34).

14. Kit (10, 30, 50) according to one or more of the claims 11 to 13, **characterized in that** the covers (30) have different colors.

15. Kit (10, 30, 50) according to one or more of the claims 10 to 14, **characterized in that** the covers (30) can be clamped onto the housings (10).

16. Kit (10, 30, 50) according to one or more of the claims 10 to 15, **characterized in that** the covers (30) can be attached to the housings (10) by means of a first latching connection.

17. Kit (10, 30, 50) according to one or more of the claims 10 to 16, **characterized in that** the covers (30) are rotatably attached onto the housing (10).

## Revendications

1. Couvercle (30) comprenant une plaque (50) pour fixation au niveau du couvercle (30) pour un boitier (10) d'un d'échangeur de chaleur-humidité pour des laryngectomisés et trachéotomisés, comportant une plaque de recouvrement réalisée en tant que disque largement circulaire, un bord de couvercle (31) et un nombre d'évidements (34) traversant le couvercle (30), la plaque de recouvrement comportant un ou plusieurs évidements, au moins un premier élément d'enclenchement (32) en partie circonférenciellement circulaire étant disposé coté intérieur au niveau du bord de couvercle (31), le bord de couvercle (31) comportant coté extérieur au moins un deuxième élément d'enclenchement (33) en partie circonférentiel, la plaque comportant une ouverture de respiration (54), la plaque (50) étant de conception en forme de trémie autour de l'ouverture de respiration (54), afin de guider un doigt sur le trou de respiration, lors d'une fermeture voulue de celui-ci, la plaque (50) comportant un logement d'enclenchement (52) au moins en partie circonférentiel coté intérieur, pour le deuxième élément d'enclenchement (33) du couvercle (30).

2. Couvercle (30) comprenant une plaque (50) selon la revendication 1, **caractérisé en ce que** le bord de couvercle (31) peut être fixé sur un boitier (10) par serrage.

3. Couvercle (30) comprenant une plaque (50) selon l'une ou plusieurs des précédentes revendications, **caractérisé en ce que** le bord du couvercle (31) comporte des éléments de serrage.

4. Couvercle (30) comprenant une plaque (50) selon l'une ou plusieurs des précédentes revendications, **caractérisé en ce que** les éléments d'enclenchement (32) sont répartis circonférenciellement au niveau de la périphérie intérieure du bord du couvercle (31).

5. Couvercle (30) comprenant une plaque (50) selon l'une ou plusieurs des précédentes revendications, **caractérisé en ce que** des évidements (34) traversent le couvercle (30) dans un angle (36) d'environ 30° à environ 60° par rapport à une normale (38) sur une surface du couvercle (40).

6. Couvercle (30) comprenant une plaque (50) selon l'une ou plusieurs des précédentes revendications, **caractérisé en ce qu'**au moins une partie des évidements (34) sont des perçages.

7. Couvercle (30) comprenant une plaque (50) selon l'une ou plusieurs des précédentes revendications, **caractérisé en ce qu'**au moins une partie des évidements (34) sont des fentes.

8. Couvercle (30) comprenant une plaque (50) selon la revendication 7, **caractérisé en ce que** des lamelles (35) sont disposées entre les fentes.

9. Couvercle (30) comprenant une plaque (50) selon la revendication 1, **caractérisé en ce que** le couvercle (30) peut être fixé au moyen d'une deuxième liaison d'enclenchement au niveau de la plaque (50).

10. Ensemble (10, 30, 50) comportant au moins un nombre de boitiers (10), les boitiers (10) comportant un logement d'enclenchement (22) pour recevoir un premier élément d'enclenchement (32) d'un couvercle (30) selon l'une ou plusieurs des revendications 1 à 9 et un nombre de couvercles (30) comportant une plaque (50) selon l'une des revendications 1 à 9.

11. Ensemble (10, 30, 50) selon la revendication 10, **caractérisé en ce que** les boitiers (10) sont des articles jetables.

12. Ensemble (10, 30, 50) selon l'une des revendications 10 ou 11, **caractérisé en ce que** les plaques (50) présentent un nombre de différentes couleurs et/ou formes extérieures.

13. Ensemble (10, 30, 50) selon l'une ou plusieurs des revendications 10 à 12, **caractérisé en ce que** les couvercles (30) comportent un nombre d'évidements (34) différemment conçus.

14. Ensemble (10, 30, 50) selon l'une ou plusieurs des revendications 11 à 13, **caractérisé en ce que** les couvercles (30) présentent des couleurs différentes.

15. Ensemble (10, 30, 50) selon l'une ou plusieurs des revendications 10 à 14, **caractérisé en ce que** les couvercles (30) peuvent être enclipsés sur les boitiers (10).

16. Ensemble (10, 30, 50) selon l'une ou plusieurs des revendications 10 à 15, **caractérisé en ce que** les couvercles (30) peuvent être fixés au moyen d'une premier liaison d'enclenchement sur les boitiers (10).

17. Ensemble (10, 30, 50) selon l'une ou plusieurs des revendications 10 à 16, **caractérisé en ce que** les couvercles (30) peuvent être tournés sur le boitier (10).
